# EUROPÄISCHE PATENTSCHRIFT

(11) **EP 0 947 219 B1**
(45) Veröffentlichungstag und Bekanntmachung des Hinweises auf die Patenterteilung: **23.01.2008**
(21) Anmeldenummer: 98122782.0
(22) Anmeldetag: 01.12.1998
(51) Int. Cl.: A61N 5/06, A61F 9/007

(54) **Anordnung zur Kontrolle und Steuerung der Behandlungsparameter an einem ophthalmologischen Behandlungsgerät**
Apparatus for monitoring and control of the treatment parameters of an ophthalmological treatment device
Appareillage de contrôle et de régulation des paramètres de traitement d'un dispositif de traitement ophthalmologique

(30) Priorität: 30.03.1998 DE 19814095
(43) Veröffentlichungstag der Anmeldung: 06.10.1999
(60) Teilanmeldung: 07006219.5
(73) Patentinhaber: Carl Zeiss Meditec AG, 07745 Jena (DE)
(72) Erfinder: Dubnack, Steffen Dipl.-Ing., 07745 Jena (DE)
(74) Vertreter: Muhsfeldt, Willi

(56) Entgegenhaltungen:
- EP-A- 0 492 778
- WO-A-84/03220
- WO-A-90/08523
- WO-A-97/17011
- DE-A- 4 227 390
- GB-A- 2 252 249
- US-A- 4 682 595
- US-A- 5 279 298
- US-A- 5 295 989

## Beschreibung

Die Erfindung betrifft eine Anordnung zur Kontrolle und Steuerung der Einstellung der das Auge therapierenden Behandlungsparameter an einem ophthalmologischen Behandlungsgerät, insbesondere zur Einstellung und Kontrolle der Applizierung der patientenbezogenen, tatsächlich zu applizierenden Strahlungsmenge (Behandlungsparameter) bei der photodynamischen Therapie (PDT) krankhafter Gewebe im Augenhintergrund, insbesondere zur Behandlung altersbedingter Makuladegeneration, welche meist zu einer (fortschreitenden) Zerstörung der zentralen Netzhautteile mit irreversiblem Verlust der Sehfunktion führt. Bei der proliferativen Form der Makuladegeneration ist eine Neovaskularisation, die ihren Ursprung in der Aderhaut hat und unter die Netzhaut des Auges zerstörend hineinwächst, für den Krankheitsverlauf verantwortlich. Im Endstadium kommt es zu schädlicher Narbenbildung. Eine Anordnung gemäß dem Oberbegriff des Anspruchs 1 ist aus WO-A-84/03220 bekannt.

So ist es bekannt, durch eine photodynamische Therapie Thrombosen und Neovaskularisationen im Auge zu isolieren, indem durch photodynamische Effekte eine selektive und lokalisierte Behandlung der betroffenen Stellen und Gewebeteile des Auges vorgenommen wird. Um eine solche Behandlung krankhafter Gewebe gezielt durchführen zu können, werden dem Patienten Photosensibilisatoren injiziert, welche sich nach einer gewissen Zeit selektiv an krankhaften Gewebeteilen des Auges anreichern und eine effektive Strahlenbehandlung mit Laserlicht geeigneter Wellenlänge ermöglichen.

Für die Photosensibilisierung nach einer Sensibilisatorinjektion werden vorzugsweise Laser verwendet, welche eine zur Anregung der Photosensibilisierung geeignete Wellenlänge einstellbarer Leistung aussenden, wobei der eingestellte Leistungswert u. a. vom injizierten Farbstoff und auch von evtl. vorhandenen Trübungen im zu behandelnden Auge abhängt.

Das vom Laser ausgesendete Licht wird über geeignete optische Elemente, z. B. Lichtleiter, in einen Applikator eingekoppelt, über den das Licht auf den zu behandelnden Abschnitt der Retina des Auges gerichtet wird. Die Einstellgrößen, wie Durchmesser des Behandlungsareals und die Strahlungsleistung werden durch den behandelnden Arzt ermittelt und eingestellt.

Ein Zielstrahl, mit dessen Hilfe der Arzt den Applikator so ausrichtet, dass ein Therapiestrahl die zu behandelnde Fläche in Auge exponieren kann, wird von einem zweiten, wesentlich schwächeren Laser erzeugt, welcher Licht einer anderen Wellenlänge aussendet. Auch dieser Zielstrahl wird durch die optischen Elemente in den Applikator eingekoppelt.

Bei den Behandlungen sind die patientenbezogenen Parameter, wie Größe des zu behandelnden Areals im Auge, die Beachtung des Einflusses von evtl. vorhandenen Trübungen im Patientenauge sowie die Einhaltung einer bestimmten maximalen Strahlungsintensität und farbstoffbezogener Parameter (Energieflussdichte) zu beachten. Bei den derzeit eingesetzten Lasersystemen können jedoch nur die Parameter, Leistung, Energie und Bestrahlungszeit am Gerät eingestellt werden. So ist es nachteilig, dass der Arzt oder Anwender zur Anpassung der tatsächlich zu verwendenden Parameter mittels Tabellen oder anderer Hilfsmittel die einzelnen Werte ineinander umrechnen und deren Einhaltung garantieren muss. Diese Methode ist eine Quelle subjektiv durch den Arzt oder Anwender bedingter Fehler, die sich unmittelbar auf den Erfolg der Behandlung auswirken.

So ist es Aufgabe der Erfindung, diese Nachteile des Standes der Technik zu beseitigen und eine Anordnung zur Kontrolle und Steuerung der Behandlungsparameter an einem ophthalmologischen Behandlungsgerät zu schaffen, mit der eine von subjektiven Einflüssen weitestgehend freie, photodynamische Therapie von erkrankten Geweben im Auge erzielbar ist und eine sichere Handhabung und Einstellung dieser Geräte ermöglicht wird.

Erfindungsgemäß wird diese Aufgabe mit einer Anordnung in einfacher Weise bei einem ophthalmologischen Gerät gelöst, wobei das Gerät eine einen Ziel- und einen Therapiestrahl erzeugende Strahlungsquelle, einen Rechner oder eine Steuereinheit und einen die Strahlung in das zu behandelnde Auge richtenden und eine Beobachtung durch den Bedienenden ermöglichenden Applikator umfasst, welcher an eine Spaltlampe ansetzbar ist. Als Strahlungsquelle kann z. B. eine Lasereinheit mit einem Ziel- und einem Therapielaser vorgesehen sein. Die Strahlungsquelle und der Applikator sind durch mindestens ein optisches Element, beispielsweise einen Lichtleiter, der den Ziel- und Therapiestrahl überträgt, miteinander verbunden. Damit auch zwischen dem Applikator und der Lasereinheit Kontaktglasvergrößerung, Energiedosis je Flächeneinheit, Intensität und Spotgröße und/oder andere, Steuergrößen repräsentierende Informationen übertragen werden können, ist eine Informationsverbindung vorgesehen. Diese kann in Form einer elektrischen Verbindung, z. B. im Form eines elektrischen Kabels, ausgebildet sein, welches z. B. an dem Teil des Applikators ankoppelbar ist, an welchem auch der Eingang des beispielsweise als Lichtleiter ausgebildeten optischen Elementes vorgesehen ist.

So wird die Intensität der Laserstrahlung am Bestrahlungsort im Auge mit der zu applizierenden Intensität der Strahlung verglichen und bei Unterschieden zwischen diesen Intensitäten wird die Intensität der Laserstrahlung automatisch oder auch manuell durch Veränderung der Spotgröße mittels eines im Applikator vorgesehenen optischen Systems und/oder durch Veränderung der Leistung der Strahlungsquelle während der Bestrahlung konstant gehalten. Das optische System umfasst ein Strahlaufweitungssystem und ein Objektiv. Um eine große Sicherheit für den Patienten zu erreichen, werden Veränderungen der Einstellung der Spotgröße mit Hilfe des optischen Systems des Applikators automatisch von der Strahlungsquelle erfaßt und ausgeregelt oder durch Abschaltung des Lasers die Bestrahlung unterbrochen bis die gewünschte (vorgesehene) Intensität der eingesetzten Strahlung wieder eingestellt ist. Die Übertragung der entsprechenden elektrischen Steuersignale erfolgt durch die Informationsverbindung zwischen der Strahlungsquelle und dem Applikator, z. B. über ein elektrisches Kabel.

Vorteilhaft bei der Anordnung ist es, wenn sie mit einem Timer ausgerüstet ist, der vom Bedienenden beliebig gesetzt werden kann und mit welchem die Zeit von der Injektion des Farbstoffes bis zum Beginn der Bestrahlung vorgebbar ist. Der Ablauf des Timers kann dem Bedienenden optisch und/oder akustisch angezeigt werden. Diese Kombination verbessert auch die vorhandene Schnittstelle Anwender/Patient und Therapiegerät und erhöht die Sicherheit für den Patienten bei der Bestrahlung und auch den Behandlungserfolg.

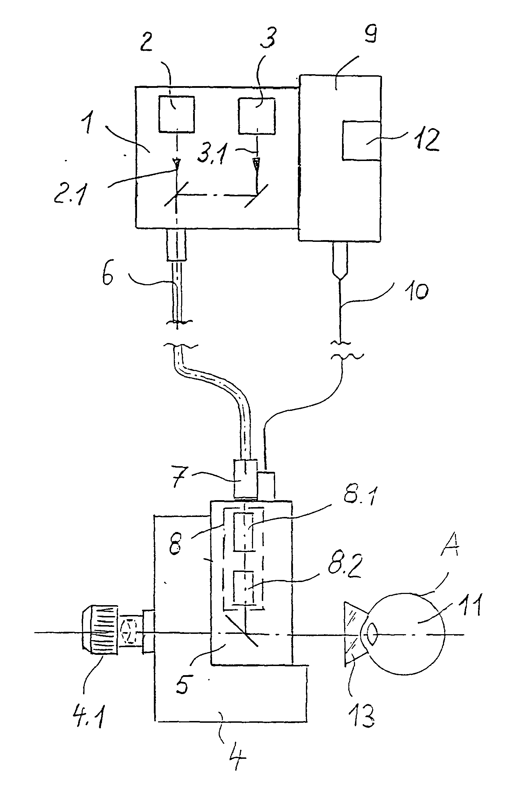

Die Erfindung soll nachstehend an einem Ausführungsbeispiel näher erläutert werden. In der Zeichnung ist schematisch eine Anordnung vereinfacht dargestellt.

Zur Kontrolle und Steuerung der Einstellung der Behandlungsparameter bei der photodynamischen Therapie krankhafter Gewebe im Innern des Auges A wird ein Behandlungsgerät benutzt, welches eine einen Therapiestrahl 3.1 und einen Zielstrahl 2.1 erzeugende Strahlungsquelle 1 mit Ziellaser 2 und Therapielaser 3 sowie Spaltleuchte 4 umfasst, an die ein sogenannter Applikator 5 ankoppelbar ist. Spaltleuchte 4 und Applikator 5 bilden in gekoppeltem Zustand die Einheit, durch welche auch der Therapie- 3.1 und Zielstrahl 2.1 zur Bestrahlung auf bzw. in das Auge gerichtet werden kann und mit welcher der behandelnde Arzt durch eine Beobachtungsoptik 4.1 das Auge A beobachten kann, auf welches zur Einkopplung des Lichtes ein Kontaktglas 13 aufgesetzt ist. Der Applikator 5 selbst ist mit der Strahlungsquelle 1 durch ein optisches Element 6 verbunden, welcher das Licht des Ziel- 2.1 und des Therapiestrahls 3.1 von der als Lasereinheit ausgebildeten Strahlungsquelle 1 zum Applikator 5 leitet. Zur Ankopplung des optischen Elementes 6 ist am Applikator 5 ein Koppler 7 mit integrierter Einkoppeloptik (nicht dargestellt) vorgesehen. Ferner ist im Applikator 5 ein optisches System 8 vorgesehen, welches ein Strahlaufweitungssystem 8.1 und ein Objektiv 8.2 oder auch nur ein Zoomobjektiv umfasst, wobei mit dem Strahlaufweitungssystem 8.1 und unter Berücksichtigung des auf dem Auge A des Patienten aufgesetzten Kontaktglases 13 die Größe des Spots und die Vergrößerung eingestellt werden kann.

Bei der Arbeit mit dem Behandlungsgerät werden an der Lasereinheit 1 die Behandlungsparameter, wie Intensität, die Vergrößerung des Kontaktglases 13, welches auf das Auge A aufgesetzt wird, und auch die Energiedosis/Flächeneinheit und die Größe des Spots durch den Bedienenden bzw. behandelnden Arzt vorgewählt und eingestellt. Anhand von Einflußgrößen, die vom Patienten abhängen bzw. von ihn beeinflusst sind, wie z. B. die auf der Retina zu realisierende Spotgröße, werden die am Applikator 5 einzustellenden Gerätesollparameter berechnet, um die für die Behandlung zu applizierende Intensität und Spotgröße des Therapiestrahls am Behandlungsort auf der Retina realisieren zu können. Durch einen Vergleich der am Applikator 5 eingestellten Parameter mit den für die Bestrahlung zu realisierenden Gerätesollparametern mittels eines Rechners 9 und der Herbeiführung einer Übereinstimmung und Konstanthaltung dieser als Sollwerte geltenden Parameter wird erreicht, dass während der Bestrahlung des zu behandelnden Auges A stets genau definierte Bedingungen bei der Bestrahlung erzielt und auch eingehalten werden und eine Über- bzw. Unterschreitung der für die Behandlung notwendigen Intensität und/oder Bestrahlungsdosis vermieden wird.

Die jeweils abzugebende Leistung der Strahlenquelle wird aus der Spotgröße auf der Retina und der eingestellten Intensität des Therapiestrahls ermittelt. Die Behandlung dauert so lange, bis die voreingestellte Strahlungsdosis (Energiedichte/Flächeneinheit) erreicht ist. Dann wird der Therapielaser 3 automatisch abgeschaltet. Während der Behandlung (Bestrahlung) des Auges A des Patienten wird die applizierte Intensität über die Bestrahlungszeit erfasst und integriert, wobei bei Unterbrechungen der Behandlung auch die Integration (Summation) der Intensität unterbrochen wird. Die noch für den Rest der Bestrahlung notwendige Zeit, also die Restzeit, wird dem behandelnden Arzt angezeigt. Es ist auch möglich, die Zeit zu registrieren, in welcher bereits eine Bestrahlung des Auges vorgenommen wurde.

Um das zuverlässig zu realisieren, wird die Intensität der am Behandlungsort im Fundus 11 des Auges A applizierten Strahlung mit der dort zu applizierenden Sollintensität verglichen und automatisch auch bei einer evtl. Veränderung der Spotgröße während der Bestrahlung konstant gehalten. Die Veränderung der Spotgröße erfolgt durch Einstellung des Strahlaufweitungssystems 8.1, welches Bestandteil des optischen Systems 8 ist, das im Applikator 5 angeordnet ist. Die Veränderungen der Einstellung der Spotgröße mittels des Strahlaufweitungssystems 8.1 werden automatisch von der Strahlungsquelle 1 erfasst. Bei Vorhandensein von Differenzen in der Einstellung der Spotgröße am Strahlaufweitungssystems 8.1 des Applikators 5 und der für die Bestrahlung notwendigen Spotgröße wird der Therapielaser 3 automatisch abgeschaltet, um eine Fehlbestrahlung zuverlässig zu verhindern. Ebenso werden die Zeit und die Integration der Intensität gestoppt. Wird dann die vorgesehene Größe des Spots wieder am Applikator eingestellt, erfolgt wieder eine Einschaltung des Therapielasers 3 und die Intensität wird wieder integriert und damit die Restzeit für die Bestrahlung weiter reduziert.

Zur Übertragung dazu notwendiger Signale, die u. a. der Steuerung des Strahlaufweitungssystems 8.1, bzw. wenn verwendet, eines Zoomobjektivs, im Applikator 5 dienen und Bestrahlungs- bzw. Behandlungsparameter und/oder Steuergrößen repräsentieren, ist zwischen der Strahlungsquelle 1 und dem Applikator 5 mindestens eine Informationen übertragende Verbindung 10, insbesondere eine elektrische Verbindung in Form eines Kabels, vorgesehen. Vorteilhaft ist dieses Kabel an dem Teil des Applikators 5 angeordnet, an welchem sich auch der Koppler 7 für das optische Element 6 befindet.

Um den behandelnden Arzt auf Abweichungen der tatsächlichen Spotgröße von der vorgesehenen Spotgröße aufmerksam zu machen und zu signalisieren, dass eine Veränderung in dieser Hinsicht am Behandlungsgerät vorgenommen werden muß, werden vorteilhaft akustische und/oder optische Signale gegeben, welche teilweise so lange andauern können, bis wieder eine Übereinstimmung der tatsächlichen mit der notwendigen Spotgröße per Hand oder automatisch hergestellt wurde. Diese Signale können auch nur während einer gewissen Zeit gegeben werden.

Insbesondere durch eine inverse Anzeige auf dem Anzeigenfeld der Gerätes können solche Abweichungen der Spotgröße angezeigt werden. Die Art der Abweichung von der vorgeschriebenen Position bzw. die Richtung der vorzunehmenden Einstellung kann in sinnvoller Weise durch Pfeile angezeigt werden. Ist dann der richtige Wert wieder eingestellt, erscheint die Anzeige wieder in normaler Darstellung.

Während der Behandlung sind somit beliebig viele und auch beliebig lange Unterbrechungen möglich, wobei jeweils die Integration der Intensität unterbrochen und der oder die die Zeit zählenden Zähler 12 gestoppt werden.

Das Behandlungsgerät kann auch mit einem Timer (Zeitzähler) versehen werden, der vom Anwender eingestellt wird und z. B. die Zeit vorgibt, die von der Injektion des Farbstoffs bis zum Beginn der Bestrahlung vergehen soll. Vorteilhafterweise sind hier Rückwärtszähler anzuwenden. Bei Ablauf der vorgeschriebenen Zeit werden in regelmäßigen Abständen akustische und/oder optische Signale generiert, die die bevorstehende Behandlung ankündigen. Durch diese Kombination werden vor allem die Schnittstelle zwischen Anwender/Patient und Behandlungsgerät verbessert und die Sicherheit bei der Bestrahlung von Teilen im Auge sowie der Behandlungserfolg erhöht.

## Patentansprüche

1. Anordnung zur Kontrolle und Steuerung der Behandlungsparameter an einem ophthalmologischen Behandlungsgerät, umfassend eine einen Therapie- (3.1) und wahlweise auch einen Zielstrahl (2.1) erzeugende Strahlungsquelle (1) mit einer Steuereinheit oder einem Rechner (9) und einen die Strahlung in das zu behandelnde Auge A richtenden und eine Beobachtung durch den Bedienenden ermöglichenden Applikator (5), welcher an einer Spaltleuchte (4) ansetzbar ist, wobei die Strahlungsquelle (1) und der Applikator (5) zur Übertragung der Strahlung durch ein optisches Element (6) verbunden sind,
**dadurch gekennzeichnet,**
**dass** an der Steuereinheit oder dem Rechner (9) mindestens ein Bestrahlungsparameter aus der Gruppe Kontaktglasvergrößerung, Energiedosis je Flächeneinheit, Intensität und Spotgröße einstellbar ist, dass zur Übertragung von Steuergrößen repräsentierenden Signalen und Informationen über diesen mindestens einen Bestrahlungsparameter,
zwischen der Strahlenquelle (1) bzw. Steuereinheit oder dem Rechner (9)
und dem Applikator (5) mindestens eine die Steuergrößen übertragende Verbindung (10) vorgesehen ist
und weiterhin ein die Zeit der Bestrahlung registrierender Zähler (12) vorgesehen ist.

2. Anordnung nach Anspruch 1, **dadurch gekennzeichnet, dass** die mindestens eine Verbindung (10) eine Informationen oder Steuergrößen übertragende elektrische Verbindung ist, welche an dem Teil des Applikators (5) ankoppelbar ist, an welchem auch der Eingang des Lichtwellenleiters (6) angeordnet ist.

3. Anordnung nach Anspruch 2, **dadurch gekennzeichnet, dass** die elektrische Verbindung ein Kabel ist, welches an dem Teil des Applikators (5) ankoppelbar ist, an welchem auch der Eingang des Lichtwellenleiters (6) angeordnet ist.

4. Anordnung nach Anspruch 1, **dadurch gekennzeichnet, dass** ein vom Bedienenden einzustellender Timer vorgesehen ist, mit welchem die Zeit von der Injektion des Farbstoffes bis zum Beginn der Bestrahlung vorgebbar ist, wobei der Ablauf des Timers optisch und/oder akustisch dem Bedienenden angezeigt wird.

5. Anordnung nach Anspruch 1, **dadurch gekennzeichnet, dass** das optische Element (6) ein Lichtleiter ist.

6. Anordnung nach Anspruch 1, **dadurch gekennzeichnet, dass** die Strahlungsquelle (1) eine Lasereinheit ist.

7. Anordnung nach Anspruch 1, **dadurch gekennzeichnet, dass** ein optisches System (8) im Applikator vorgesehen ist, welches ein Strahlaufweitungssystem (8.1) und ein Objektiv (8.2) umfasst.

## Claims

1. Arrangement for monitoring and controlling the treatment parameters of an ophthalmological treatment apparatus, comprising a radiation source (1) producing a treatment beam (3.1) and selectively also a target beam (2.1) and having a control unit or a computer (9) and an applicator (5) directing radiation into the eye A to be treated and permitting monitoring by the operator and being attachable to a slit lamp (4), wherein the radiation source (1) and the applicator (5) are connected by an optical element (6) in order to transmit the radiation,
**characterised in**
**that** at least one radiation parameter from the group consisting of contact lens magnification, energy dose per unit of area, intensity and spot size, can be adjusted on the control unit or on the computer (9),
**that** in order to transmit signals representing control variables and information about this at least one radiation parameter between the beam source (1) or the control unit or the computer (9) and the applicator (5) at least one connection (10) transmitting the control variables is provided and that furthermore a counter (12) recording the radiation time is provided.

2. Arrangement as claimed in claim 1, **characterised in that** the at least one connection (10) is an electrical connection transmitting information or control variables and able to be coupled to the part of the applicator (5) on which the input of the light wave conductor (6) is also disposed.

3. Arrangement as claimed in claim 2, **characterised in that** the electrical connection is a cable which can be coupled to the part of the applicator (5) on which the input of the light wave conductor (6) is also disposed.

4. Arrangement as claimed in claim 1, **characterised in that** a timer to be set by the operator is provided, with which the time from the injection of the dye to the commencement of the radiation can be preset, wherein the progress of the timer is indicated to the operator visually and/or acoustically.

5. Arrangement as claimed in claim 1, **characterised in that** the optical element (6) is a light conductor.

6. Arrangement as claimed in claim 1, **characterised in that** the radiation source (1) is a laser unit.

7. Arrangement as claimed in claim 1, **characterised in that** an optical system (8) is 10 provided in the applicator and includes a beam-expanding system (8.1) and an objective (8.2).

## Revendications

1. Dispositif pour le contrôle et la commande des paramètres de traitement sur un appareil de traitement ophtalmologique, comprenant une source de rayonnement (1) générant un faisceau de thérapie (3.1) et en option également un faisceau cible (2.1) avec une unité de commande ou un ordinateur (9) et un applicateur (5) dirigeant le rayonnement dans l'oeil A à traiter et une observation par l'opérateur, lequel applicateur peut être placé sur une lampe à fente (4), la source de rayonnement (1) et l'applicateur (5) pour la transmission du rayonnement étant reliés par un élément (6) optique,
**caractérisé en ce que**,
au moins un paramètre d'exposition au rayonnement provenant du groupe agrandissement de verre de contact, dose d'énergie par unité de surface, intensité et grandeur du spot peut être réglé sur l'unité de commande ou l'ordinateur (9),
**en ce que**, pour la transmission de signaux représentant des grandeurs de commande et d'informations relatives à ce au moins un paramètre d'exposition au rayonnement, il est prévu au moins une liaison (10) transmettant les grandeurs de commande entre la source de faisceau (1) respectivement l'unité de commande ou l'ordinateur (9) et l'applicateur (5) et un compteur (12) enregistrant le temps de l'exposition au rayonnements est également prévu.

2. Dispositif selon la revendication 1, **caractérisé en ce qu'**au moins une liaison (10) est une liaison électrique transmettant des informations ou des grandeurs de commande, laquelle liaison peut être rattachée à la partie de l'applicateur (5) sur laquelle est disposée également l'entrée du guide d'ondes optiques (6).

3. Dispositif selon la revendication 2, **caractérisé en ce que** la liaison électrique est un câble qui peut être rattaché à la partie de l'applicateur (5) sur laquelle est disposée également l'entrée de guide d'ondes optiques (6).

4. Dispositif selon la revendication 1, **caractérisé en ce qu'**une minuterie à régler par l'opérateur est prévue, avec laquelle le temps s'écoulant depuis l'injection du colorant jusqu'au début de l'exposition au rayonnement peut être prédéfini, le déroulement de la minuterie pouvant être affiché de façon visuelle et/ou acoustique pour l'opérateur.

5. Dispositif selon la revendication 1, **caractérisé en ce que** l'élément (6) optique est un guide d'ondes.

6. Dispositif selon la revendication 1, **caractérisé en ce que** la source de rayonnement (1) est une unité laser.

7. Dispositif selon la revendication 1, **caractérisé en ce qu'**un système (8) optique est prévu dans l'applicateur, lequel comprend un système d'élargissement de faisceau (8.1) et un objectif (8.2).
